# FASCICULE DE BREVET EUROPEEN

(11) **EP 2 498 734 B1**
(45) Date de publication et mention de la délivrance du brevet: **30.05.2018**
(21) Numéro de dépôt: 10773092.1
(22) Date de dépôt: 03.11.2010
(51) Int. Cl.: A61F 6/00, B65D 75/58

(54) **EMBALLAGE POUR PRESERVATIF**
KONDOMVERPACKUNG
CONDOM WRAPPING

(30) Priorité: 13.11.2009 LU 91622
(43) Date de publication de la demande: 19.09.2012
(73) Titulaire: Sweetgum SARL, 1724 Luxembourg (LU)
(72) Inventeur: DE WALEFFE, Xavier, 6110 Montigny-le-Tilleul (BE)
(74) Mandataire: Lecomte & Partners
(86) Numéro de dépôt international: PCT/EP2010/066755
(87) Numéro de publication internationale: WO 2011/057931

(56) Documents cités:
- DE-U1- 20 215 481
- JP-U- 51 155 277
- JP-U- 62 125 521
- JP-U- 63 133 210
- JP-U- 63 195 822
- US-A- 4 875 491

## Description

L'invention a trait à un emballage de préservatif selon le préambule de la revendication 1 (cf. JP 62-125521 U). Pour diverses raisons d'hygiène et de sécurité, les préservatifs sont emballés de manière individuelle. Ces emballages comprennent typiquement deux feuillets de film souple superposés et soudés entre eux à leurs bords de manière à être hermétiques. Le préservatif y est logé dans un état roulé avec le réservoir en regard voire en contact avec la face interne d'un des deux feuillets. Ce type d'emballage comprend habituellement une entaille pratiquée sur un bord, de manière à permettre à son utilisateur d'amorcer aisément la déchirure qui permet d'ouvrir l'emballage et en extraire le préservatif. La plupart de ces emballages ne permettent pas d'identifier le sens du préservatif au moment de la sortie de ce dernier de l'emballage. En effet, ces emballages sont symétriques, à savoir que les faces extérieures des feuillets sont identiques et ne permettent pas d'identifier avant ouverture le sens du préservatif.

Un préservatif a en effet un sens dans la mesure où il est emballé dans un état roulé dans un sens donné depuis le bord inférieur jusqu'au réservoir. Bien qu'il soit réversible et puisse théoriquement être utilisé dans un sens comme dans l'autre, il est cependant impératif de le disposer dans le bon sens au sommet du pénis en vue de pouvoir le dérouler jusqu'à la base de manière inverse au sens dans lequel il a été enroulé. Dans son état roulé, le préservatif présente une forme annulaire correspondant à l'enroulement de la partie cylindrique, avec le réservoir disposé au centre de cette partie annulaire. En raison de l'extrême élasticité du préservatif, le réservoir peut se retourner très facilement lors de sa manipulation, c'est-à-dire être dirigé indistinctement dans l'un ou l'autre sens dans une direction perpendiculaire au plan général de la partie annulaire. Il est donc assez difficile d'identifier le bon sens du préservatif une fois celui-ci pris en main par la partie annulaire, en particulier dans l'obscurité. Une tentative de mise en place du préservatif dans le mauvais sens le rend inutilisable ou alors entraine un risque de contamination du fait qu'il doit être retourné après un premier contact avec le pénis. Diverses solutions ont été développées pour contrer cette difficulté.

Le document de brevet WO 98/46495 prévoit une ligne d'affaiblissement médiane au niveau des deux feuillets constituant l'emballage ainsi que des petites boucles disposées sur la face extérieure d'un des deux feuillets et au niveau de deux bords opposés et parallèles à la ligne médiane d'affaiblissement. De la sorte, l'utilisateur peut, théoriquement d'une seule main, ouvrir l'emballage, c'est-à-dire par un mouvement de fermeture de la main destinée à démarrer la déchirure au niveau de la ligne médiane et ensuite par un mouvement d'extension du pouce de la main qui engage une des boucles, générant ainsi un effort de séparation des deux parties séparées par la ligne médiane. Au vu de la présence des boucles sur une seule face, l'ouverture se fait alors théoriquement toujours du même côté du préservatif, ce qui constitue un repère pour l'utilisateur. Cette solution présente cependant deux inconvénients majeurs, à savoir le mouvement de la main qui est assez complexe, requérant une certaine dextérité et le fait que la préhension du préservatif qui se fait par la partie annulaire n'empêche pas l'utilisateur de « perdre » le sens du préservatif une fois celui-ci sorti de la moitié d'emballage restant dans le creux de la main.

Le document de brevet DE 200 14 263 U1 aborde la problématique sus mentionnée et propose comme solution un emballage équipé d'une cartouche d'air sous pression avec une sortie dirigée vers l'intérieur du réservoir de manière à le faire sortir dans le bon sens de manière franche lors de l'ouverture de l'emballage. Cet emballage bien qu'intéressant est de construction complexe et coûteuse.

Le document de brevet EP 0 604 675 A1 aborde également la problématique sus mentionnée et propose comme solution un emballage comprenant une série de petits rubans répartis sur la périphérie extérieure de la partie annulaire du préservatif à l'état roulé, ces rubans étant enroulés avec le préservatif et attachés à l'emballage. La mise en place du préservatif se fait en présentant l'emballage au sommet du pénis et en exerçant un effort sur la face inférieure de l'emballage par un mouvement correspondant de l'emballage. Les lignes d'affaiblissement sur les faces inférieures et supérieures cèdent sous la pression de manière à former une ouverture pour le pénis et le préservatif se fait enrouler automatique par le maintien du mouvement de remballage et grâce aux rubans qui assistent le déroulement de la partie annulaire. Cette solution semble certes efficace mais assez complexe et coûteuse et présente de plus un inconvénient majeur d'un point vue confort et hygiène.

Le document de brevet JP 51 155277 U divulgue un emballage de préservatif dans lequel la forme de la ligne d'affaiblissement formant la zone d'ouverture, bien qu'apte à potentiellement maintenir le préservatif dans l'emballage après ouverture, présente toutefois l'inconvénient de requérir un certain effort de manipulation pour en extraire le préservatif, à savoir soit une flexion de l'emballage ou encore un mouvement de translation du préservatif.

L'invention a pour objet de proposer un emballage répondant à la problématique sus mentionnée et palliant au moins un des inconvénients sus mentionnés.

L'invention consiste en un emballage de préservatif comprenant un préservatif et deux sections de film superposées et reliées entre elles au niveau de leurs bords de manière à former un volume de logement du préservatif dans un état roulé avec le réservoir du préservatif dans sa position normale en regard d'une des sections de film et le sens normal de déroulement dirigé vers l'autre des sections de film, la section de film en regard du réservoir comprenant une première ligne d'affaiblissement en forme générale de contour correspondant approximativement au contour du préservatif et définissant une zone d'ouverture; et des moyens de préhension de la partie de film délimitée par la ligne d'affaiblissement; de manière à ce que ladite partie de film puisse être déchirée en vue d'ouvrir remballage du côté réservoir du préservatif, le contour de la première ligne d'affaiblissement et le contour du préservatif étant généralement circulaires et le diamètre du contour de la ligne étant inférieur à celui du contour du préservatif de manière à assurer un maintien du préservatif dans l'emballage une fois ouvert tout en permettant une extraction aisée par traction sur le réservoir et sans le dérouler. L'invention permet de le déballer sans stress, également dans l'obscurité, et de prendre le préservatif dans le bon sens avec certitude, sans risque d'inversion, sans risque de contact contaminant de l'extérieur du préservatif, sans risque de chute hors de l'emballages pendant le déballage, sans risque de déchirement du préservatif avec les dents ou un objet coupant lors du déballage,

Selon un mode avantageux de l'invention, le diamètre du contour de la ligne d'affaiblissement est inférieur de 1 à 10% à celui du contour du préservatif.

Selon un autre mode avantageux de l'invention, la section de film en regard du réservoir comprend au moins une seconde ligne d'affaiblissement s'étendant depuis la première ligne d'affaiblissement vers l'extérieur de la zone d'ouverture, dirigée généralement selon un rayon du contour circulaire de la première ligne d'affaiblissement. Ces lignes optionnelles permettent une extraction plus facile du préservatif.

Selon un encore autre mode avantageux de l'invention, la section de film en regard du réservoir comprend plusieurs, préférentiellement quatre, secondes lignes d'affaiblissement, réparties uniformément sur le contour de la première ligne d'affaiblissement.

Selon un encore autre mode avantageux de l'invention, les moyens de préhension sont tels qu'ils permettent une reconnaissance tactile de la section de film en regard du réservoir. Le déballage du préservatif peut ainsi se réaliser dans l'obscurité totale, grâce à une reconnaissance tactile infaillible.

Selon un encore autre mode avantageux de l'invention, les moyens de préhension comprennent un marquage visible dans l'obscurité, préférentiellement un marquage phosphorescent. Le déballage du préservatif peut ainsi se réaliser dans l'obscurité totale, grâce à une reconnaissance visible.

Selon un encore autre mode avantageux de l'invention, les moyens de préhension comprennent une tirette liée à la partie de film délimitée par la ligne d'affaiblissement, la tirette étant préférentiellement liée à la partie de film au niveau de la ligne d'affaiblissement de manière à y maximiser les contraintes lors d'un effort de traction exercé sur la tirette.

Selon un encore autre mode avantageux de l'invention, la tirette est liée à la partie de film délimitée par la ligne d'affaiblissement par collage ou soudage.

Selon un encore autre mode avantageux de l'invention, la tirette est une portion de film similaire au film des sections constituant l'emballage.

Selon un encore autre mode avantageux de l'invention, la première ligne et/ou la ou les secondes lignes d'affaiblissement sont pratiqués par au moins l'un des procédés suivants : fraisage, affaiblissement par rayon laser, perforation, découpage, traitement chimique.

Selon un encore autre mode avantageux de l'invention, le contour de la première ligne d'affaiblissement est ouvert de sorte à maintenir la partie de film délimitée par la ligne d'affaiblissement liée au reste de la section de film après ouverture. Cette mesure a pour effet que l'emballage reste unitaire, c'est-à-dire ne constitue qu'un seul déchet, ce qui diminue le risque de dispersion de petits déchets.

Selon un encore autre mode avantageux de l'invention, le contour de la première ligne d'affaiblissement est fermé de sorte à assurer une séparation de la partie de film délimitée par la ligne d'affaiblissement après ouverture.

D'autres caractéristiques et avantages de la présente invention seront mieux compris à l'aide de la description et des dessins parmi lesquels :
La figure 1 est une vue en perspective d'un emballage pour préservatif selon l'invention ;
La figure 2 illustre une première étape de l'opération d'ouverture de l'emballage selon la figure 1.
La figure 3 illustre une deuxième étape de l'opération d'ouverture de l'emballage selon la figure 1.
La figure 4 illustre une troisième étape de l'opération d'ouverture de l'emballage selon la figure 1.

L'enveloppe de l'emballage 2 est formée de deux feuillets composites 4 et 6 soudés, enserrant le préservatif 12 enroulé et lubrifié. Les feuillets 4 et 6 sont en principe identiques d'un point de vue forme et matériau. Le feuillet supérieur 4 se distingue cependant par les points suivants :
- pré perforation 8 d'une ouverture pour le déballage ;
- tirette 10, permettant à la fois
   ∘ le repérage du préservatif emballé dans l'environnement obscur, par un dispositif visuel;
   ∘ la détection facile et préalable du sens du préservatif par un dispositif tactile facile ;
   ∘ l'ouverture facile de l'emballage,
- marquage phosphorescent (optionnel).

A l'intérieur de l'emballage, la partie extérieure du réservoir du préservatif roulé est orientée vers le feuillet 4 équipé des signes distinctifs cités ci-dessus.

L'emballage s'ouvre exclusivement et facilement par le "décapsulage" d'une portion de la face distinguible.

Cette face est munie de pré perforations 8 permettant une découpe suivant un tracé déterminé.

Le tracé de la découpe est un cercle presque complet dont le diamètre est légèrement inférieur au diamètre du préservatif roulé.

Dans les quatre angles, des prédécoupes 14 sont amorcées dans les diagonales, partant de l'extrémité de l'emballage vers le bord du cercle prédécoupé.

Sur le cercle prédécoupé 8 est accroché (par collage ou soudure) une tirette solide 10, suffisamment grande que pour être facilement préhensible entre le pouce et l'index, et dont l'extrémité est éventuellement réticulée, et éventuellement imprimée d'un marquage phosphorescent.

La tirette 10 est constituée d'un feuillet composite d'aluminium et de plastique, indéchirable par sa forme.

La soudure ou le collage de la tirette à une résistance à la rupture supérieure à la résistance résiduelle à la rupture de la feuille multi-plis du côté prédécoupé de la pochette (voir plus bas).

La tirette est positionnée entre le centre du cercle prédécoupé et sa circonférence, voire au bord de la circonférence exclusivement, soit à la place la mieux appropriée pour assurer le déchirement de la prédécoupe de manière optimale lors de la traction entre le pouce et l'index.

A l'opposé du bord de la circonférence équipé de cette tirette, ou du bord le plus proche de cette tirette si celle ci est rapprochée du centre, le cercle n'est pas prédécoupé sur environ un quart de la circonférence, entre les deux amorces de diagonales.

En tenant l'emballage par le bord, que l'on soit gaucher ou droitier, entre le pouce et l'index d'une main, on repère très facilement, y compris dans le noir et même exclusivement de manière tactile, la tirette appliquée sur une des faces.

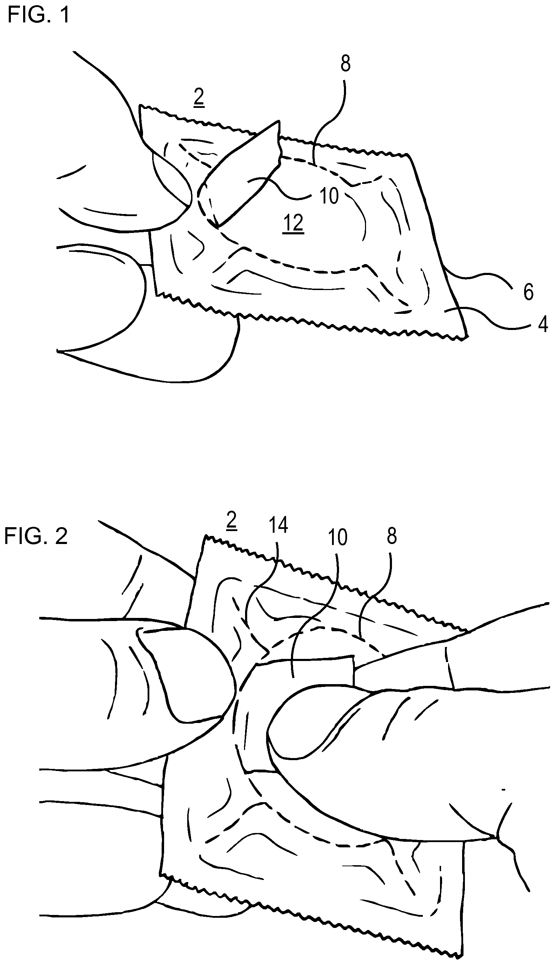

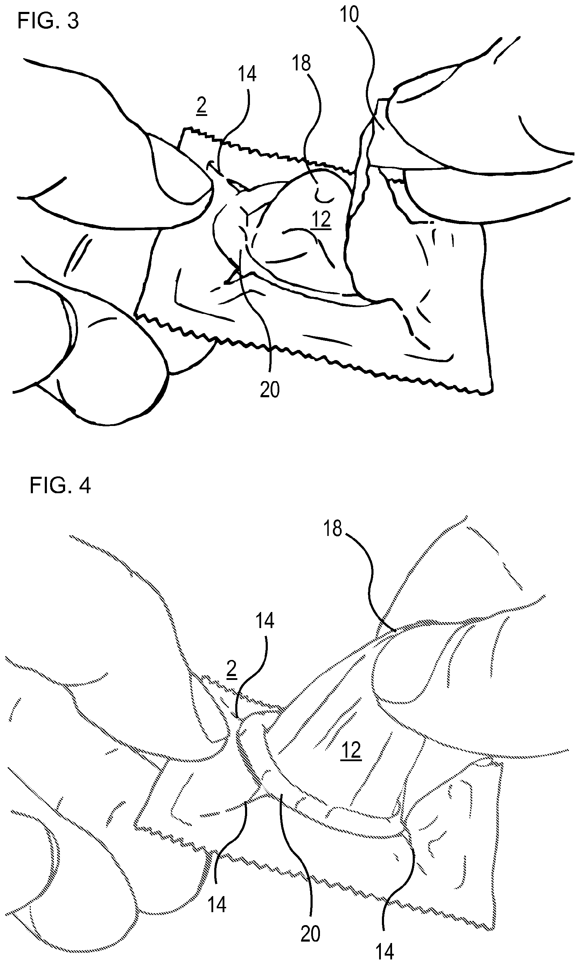

A partir du moment ou on peut tenir entre les pouces et index de chaque main le bord de l'emballage d'une part et la tirette préhensible d'autre part, il est assuré que le préservatif 12 déballé sera dans le bon sens, c'est à dire que le réservoir 18 sera au dessus lorsque l'emballage sera ouvert. Pour ouvrir, il faut simplement tirer en sens opposé les deux mains tel qu'illustré à la figure 2, et le feuillet supérieur se découpe suivant le tracé du cercle tel qu'illustré à la figure 3. Le préservatif est découvert, réservoir 18 en haut. Le préservatif ne tombe pas de l'emballage, car le diamètre de l'ouverture est très légèrement inférieur au diamètre 20 du préservatif roulé. Pour retirer le préservatif, pendant qu'une main maintient l'emballage, l'autre lâche la tirette et saisit le préservatif par le réservoir, tel qu'illustré à la figure 4. Jusqu'à cette étape, le préservatif reste légèrement maintenu dans l'emballage et ne peut pas en tomber, ni glisser. Lorsque le préservatif est saisi par le réservoir 18, il reste juste à tirer, et les diagonales prédécoupées 14 peuvent céder, de manière à libérer le préservatif uniquement si il est tenu par le réservoir. L'élasticité du préservatif roulé lui permet aussi d'être retiré de l'emballage en se déformant légèrement lorsqu'on le tire en travers des quatre amorces de diagonales, si ces amorces résistent.

L'ensemble des matériaux, sont des feuilles multi-plis d'aluminium et de plastique laminées et soudées. Le nombre et l'épaisseur des matériaux composant ce feuillet sont définis de manière à ce que l'étanchéité complète de la pochette reste garantie pour tous les agents agressifs, notamment l'étanchéité aux U.V. et à l'air, ou le déchirement accidentel par frottement lors du transport dans les vêtements, par exemple. La résistance de la soudure des deux faces de l'enveloppe doit être supérieure à la résistance résiduelle à la rupture de la feuille multi-plis du côté prédécoupé. La prédécoupe du feuillet doit se faire de manière à guider assurément la rupture de l'emballage suivant la découpe voulue, et sans création de bavures ou de résidus de parties déchirées potentiellement coupantes pour le préservatif. La prédécoupe permet d'obtenir deux entités aux bords parfaitement découpés, sans détachement de matière qui tomberait sur le préservatif ou ailleurs. La prédécoupe entame les feuillets supérieurs de manière à assurer la déchirure à l'endroit voulu, mais n'entame en aucun cas les feuillets inférieurs, ni ceux qui assurent l'étanchéité à l'air et la lumière et la solidité physique de la feuille, de manière à pouvoir garantir que l'enveloppe de protection est de qualité égale à une enveloppe réalisée sans prédécoupe. La prédécoupe est telle qu'elle entraînera à coup sûr la déchirure du feuillet suivant le tracé. La découpe sera propre, sans bavure, sans résidu, complète et en une seule manoeuvre. La prédécoupe du cercle n'est pas complète sur le diamètre, de manière à ce que la partie "capsule" ne se détache pas complètement, et que l'emballage usagé ne forme qu'un seul déchet.

L'invention présente les avantages suivants :
a) Possibilité de ne jamais se tromper de sens lors de la préhension
b) Possibilité d'ouvrir en une seule manoeuvre simple, connue et très répandue.
c) Possibilité de ne jamais se tromper de sens lors de la préhension du préservatif qui se fait exclusivement par le réservoir mis dans le bon sens.
d) Possibilité de ne jamais laisser glisser le préservatif hors de l'emballage pendant le déballage.
e) Le tout est réalisable dans le noir complet, le repérage du bon côté étant possible de manière exclusivement tactile.
f) L'emballage présente une coté moins rébarbatif, plus ludique et favorise l'emploi du préservatif, ce qui est bon tant du point de vue du fabricant (vente) que de l'effet sur la santé publique.
g) Augmentation du confort pratique : Raccourcissement du délai de déballage.
h) Augmentation du confort pratique : Certitude d'obtenir le préservatif dans le bon sens dès l'ouverture, sans aucune recherche ni manipulation nécessaire.
i) Augmentation du confort pratique et psychologique : Diminution du stress psychologique due à la certitude de placer le préservatif dans le bon sens. Il est désormais possible d'utiliser le préservatif rapidement, sans devoir rechercher le sens, et donc sans risque de le mettre en contact dans le mauvais sens puis dans le bon sens, ce qui à pour effet possible de contaminer les deux cotés du préservatif.
j) Augmentation du confort pratique : Disparition de l'effet de distraction, de gêne, de stress occasionnés par les emballages qui ne s'ouvrent pas facilement, par les petits déchets d'emballage qui se détachent et s'éparpillent, par les préservatifs qui glissent et échappent des mains, sans qu'on en retrouve le sens dans le noir.
k) Augmentation du confort psychologique : Disparition du stress occasioné par les emballages qu'on en vient parfois à essayer d'ouvrir avec les dent, au risque d'abîmer le préservatif.
l) Augmentation du confort pratique : Le fait de placer un tag ou repère fluorescent visible dans le noir sur la tirette de l'emballage rend non seulement possible de trouver le préservatif dans le bons sens lors de l'ouverture, mais aussi permet de trouver sans lumière le préservatif emballé à proximité du lieu d'utilisation, et diminue l'éventuel temps de recherche et de tâtonnement dans le noir, ce qui est rassurant.

## Revendications

1. Emballage de préservatif comprenant un préservatif (12) de contour généralement circulaire et deux sections (4, 6) de film superposées et reliées entre elles au niveau de leurs bords de manière à former un volume de logement du préservatif (12) dans un état roulé avec le réservoir (18) du préservatif dans sa position normale en regard d'une des sections de film et le sens normal de déroulement dirigé vers l'autre des sections de film,
la section de film en regard du réservoir (18) comprend :
- une première ligne d'affaiblissement (8) définissant une zone d'ouverture ; et
- des moyens de préhension (10) de la partie de film délimitée par la ligne d'affaiblissement (8) ;
de manière à ce que ladite partie de film puisse être déchirée en vue d'ouvrir l'emballage du côté réservoir du préservatif ;
**caractérisé en ce que**
la première ligne d'affaiblissement (8) est en forme générale de contour correspondant approximativement au contour du préservatif, le contour de la première ligne d'affaiblissement (8) est généralement circulaire et le diamètre du contour de ladite première ligne est inférieur à celui du contour du préservatif de manière à assurer un maintien du préservatif (12) dans l'emballage (2) une fois ouvert tout en permettant une extraction aisée par traction sur le réservoir et sans le dérouler.

2. Emballage de préservatif selon la revendication précédente, **caractérisé en ce que** le diamètre du contour de la première ligne d'affaiblissement est inférieur de 1 à 10% à celui du contour du préservatif.

3. Emballage de préservatif selon l'une des revendications précédentes, **caractérisé en ce que** la section de film (4) en regard du réservoir comprend au moins une seconde ligne d'affaiblissement (14) s'étendant depuis la première ligne d'affaiblissement vers l'extérieur de la zone d'ouverture, dirigée généralement selon un rayon du contour circulaire de la première ligne d'affaiblissement (8).

4. Emballage de préservatif selon la revendication précédente, **caractérisé en ce que** la section de film (4) en regard du réservoir comprend plusieurs, préférentiellement quatre, secondes lignes d'affaiblissement (14), réparties uniformément sur le contour de la première ligne d'affaiblissement.

5. Emballage de préservatif selon l'une des revendications précédentes, **caractérisé en ce que** les moyens de préhension (10) sont tels qu'ils permettent une reconnaissance tactile de la section de film en regard du réservoir.

6. Emballage de préservatif selon l'une des revendications précédentes, **caractérisé en ce que** les moyens de préhension (10) comprennent un marquage visible dans l'obscurité, préférentiellement un marquage phosphorescent.

7. Emballage de préservatif selon l'une des revendications précédentes, **caractérisé en ce que** les moyens de préhension comprennent une tirette (10) liée à la partie de film délimitée par la ligne d'affaiblissement (8), la tirette (10) étant préférentiellement liée à la partie de film au niveau de la ligne d'affaiblissement de manière à y maximiser les contraintes lors d'un effort de traction exercé sur la tirette.

8. Emballage de préservatif selon la revendication précédente, **caractérisé en ce que** la tirette (10) est liée à la partie de film délimitée par la ligne d'affaiblissement (8) par collage ou soudage.

9. Emballage de préservatif selon l'une des revendications 7 et 8, **caractérisé en ce que** la tirette (10) est une portion de film similaire au film des sections constituant l'emballage.

10. Emballage de préservatif selon l'une des revendications précédentes, **caractérisé en ce que** la première ligne (8) et/ou la ou les secondes lignes d'affaiblissement (14) sont pratiqués par au moins l'un des procédés suivants : fraisage, affaiblissement par rayon laser, perforation, découpage, traitement chimique.

11. Emballage de préservatif selon l'une des revendications précédentes, **caractérisé en ce que** le contour de la première ligne d'affaiblissement (8) est ouvert de sorte à maintenir la partie de film délimitée par la ligne d'affaiblissement liée au reste de la section de film après ouverture.

12. Emballage de préservatif selon l'une des revendications 1 à 10, **caractérisé en ce que** le contour de la première ligne d'affaiblissement (8) est fermé de sorte à assurer une séparation de la partie de film délimitée par la ligne d'affaiblissement après ouverture.

## Patentansprüche

1. Kondomverpackung, umfassend ein Kondom (12) mit einer allgemein kreisförmigen Kontur, und zwei übereinandergelegte Folienabschnitte (4, 6), die an den Rändern zusammengefügt sind, sodass ein Behälter zum Enthalten des Kondoms (12) in seinem aufgerollten Zustand mit dem Kondomreservoir (18) in seiner normalen Position benachbart zu einem der Folienabschnitte gebildet wird, wobei die normale Abrollrichtung des Kondoms zu dem anderen Folienabschnitt hin ist, wobei der Folienabschnitt benachbart zu dem Reservoir (18) aufweiset:
eine erste Schwächungslinie (8), die einen Öffnungsbereich umreißt; und
Mittel zum Ergreifen (10) des von der Schwächungslinie (8) umrissenen Folienteils;
derart, dass dieser Folienteil aufgerissen werden kann, um die Verpackung von der Seite des Kondomreservoirs herzu öffnen;
**dadurch gekennzeichnet, dass**
die erste Schwächungslinie (8) in einer generellen Form einer Kontur vorliegt, die annähernd der Kontur des Kondoms entspricht, die Kontur der ersten Schwächungslinie generell kreisförmig ist und der Durchmesser der Kontur der ersten Linie kleiner als derjenige der Kontur des Kondoms ist, um das Zurückhalten des Kondoms (12) in der Verpackung (2) nach dem Öffnen sicherzustellen, während ein einfaches Herausziehen davon, ohne es abzurollen, durch Ziehen an dem Reservoir gestattet wird.

2. Kondomverpackung nach dem vorhergehenden Anspruch, **dadurch gekennzeichnet, dass** der Durchmesser der Kontur der ersten Schwächungslinie 1-10% kleiner als derjenige der Kontur des Kondoms ist.

3. Kondomverpackung nach dem vorhergehenden Anspruch, **dadurch gekennzeichnet, dass** der Folienabschnitt (4) benachbart zu dem Reservoir mindestens eine zweite Schwächungslinie (14) umfasst, die sich von der ersten Schwächungslinie von dem Öffnungsbereich weg erstreckt, wobei sie generell von der kreisförmigen Kontur der ersten Schwächungslinie ausgeht.

4. Kondomverpackung nach dem vorhergehenden Anspruch, **dadurch gekennzeichnet, dass** der Folienabschnitt (4) benachbart zu dem Reservoir mehrere, bevorzugt vier, zweite Schwächungslinien (14) umfasst, die gleichmäßig auf der Kontur der ersten Schwächungslinie verteilt sind.

5. Kondomverpackung nach einem der vorhergehenden Ansprüche, **dadurch gekennzeichnet, dass** die Mittel zum Ergreifen (10) derart sind, dass sie die Identifikation durch Berühren des Folienabschnitts benachbart zu dem Reservoir zulassen.

6. Kondomverpackung nach einem der vorhergehenden Ansprüche, **dadurch gekennzeichnet, dass** die Mittel zum Ergreifen (10) Markierungen umfassen, die im Dunkeln sichtbar, bevorzugt phosphoreszierend, sind.

7. Kondomverpackung nach einem der vorhergehenden Ansprüche, **dadurch gekennzeichnet, dass** die Mittel zum Ergreifen eine Lasche (10) umfassen, die mit dem von der ersten Schwächungslinie umrissenen Folienteil verbunden ist, wobei die Lasche (10) bevorzugt mit dem Folienteil in Höhe der Schwächungslinie verbunden ist, um die Beanspruchungen während des Ziehens an der Lasche zu optimieren.

8. Kondomverpackung nach dem vorhergehenden Anspruch, **dadurch gekennzeichnet, dass** die Lasche (10) durch Kleben oder Schweißen mit dem von der ersten Schwächungslinie (8) umrissenen Folienteil verbunden ist.

9. Kondomverpackung nach Ansprüchen 7 oder 8, **dadurch gekennzeichnet, dass** die Lasche (10) ein Stück Folie ist, gleichartig zu der Folie, woraus der Rest der Verpackung hergestellt ist.

10. Kondomverpackung nach einem der vorhergehenden Ansprüche, **dadurch gekennzeichnet, dass** die erste Schwächungslinie (8) und/oder die zweite(n) Schwächungslinie(n) (14) mittels eines der folgenden Verfahren erzeugt werden: Fräsen, Schwächen durch Laserstrahl, Perforation, Schneiden, chemische Behandlung.

11. Kondomverpackung nach einem der vorhergehenden Ansprüche, **dadurch gekennzeichnet, dass** die Kontur der ersten Schwächungslinie (8) unvollständig ist, um den von der ersten Schwächungslinie umrissenen Folienteil nach dem Öffnen am Rest der Verpackung befestigt zurückzuhalten.

12. Kondomverpackung nach Ansprüchen 1 bis 10, **dadurch gekennzeichnet, dass** die Kontur der ersten Schwächungslinie (8) vollständig ist, um das Abtrennen des von der ersten Schwächungslinie umrissenen Folienteils nach dem Öffnen sicherzustellen.

## Claims

1. Condom wrapping comprising a condom (12) with a generally circular contour, and two superposed sections (4, 6) of film joined at the edges to form a container to hold the condom (12) in its rolled-up state with the condom vessel (18) in its normal position adjacent to one of the sections of film, the normal direction of unrolling the condom being towards the other section of film, the section of film adjacent to the vessel (18) having
- a first line of weakness (8) outlining an opening area; and
- means of gripping (10) the part of film outlined by the line of weakness (8);
in such a way that said part of film can be torn in order to open the wrapping from the side of the condom vessel;
**characterised in that**
the first line of weakness (8) is in a general shape of a contour corresponding approximately to the contour of the condom, the contour of the first line of weakness is generally circular and the diameter of the contour of said first line is smaller than that of the contour of the condom, in such a way as to ensure the retention of the condom (12) in the wrapping (2) after opening whilst permitting its easy extraction, without unrolling, by drawing on the vessel.

2. Condom wrapping as in the preceding claim, **characterised in that** the diameter of the contour of the first line of weakness is 1-10% smaller than that of the contour of the condom.

3. Condom wrapping as in the preceding claim, **characterised in that** the section of film (4) adjacent to the vessel includes at least one second line of weakness (14) extending from the first line of weakness away from the opening area, radiating generally from the circular contour of the first line of weakness.

4. Condom wrapping as in the preceding claim, **characterised in that** the section of film (4) adjacent to the vessel includes several, preferably four, second lines of weakness (14) distributed evenly on the contour of the first line of weakness.

5. Condom wrapping according to one of the preceding claims **characterised in that** the means of gripping (10) are such that they permit the identification by touch of the section of film adjacent to the vessel.

6. Condom wrapping according to one of the preceding claims **characterised in that** the means of gripping (10) include markings visible in the dark, preferably phosphorescent.

7. Condom wrapping according to one of the preceding claims **characterised in that** the means of gripping include a tab (10) connected to the part of the film outlined by the first line of weakness, the tab (10) preferably being connected to that part of the film at the level of the line of weakness, so as to optimise stresses during the pulling of the tab.

8. Condom wrapping as in the preceding claim, **characterised in that** the tab (10) is connected to the part of the film outlined by the first line of weakness (8) by gluing or soldering.

9. Condom wrapping as in claims 7 or 8, **characterised in that** the tab (10) is a piece of film similar to the film of which the rest of the wrapping is made.

10. Condom wrapping according to one of the preceding claims, **characterised in that** the first line of weakness (8) and/or the second line(s) of weakness (14) are created by means of one of the following processes: milling, weakening by laser beam, perforation, cutting, chemical treatment.

11. Condom wrapping according to one of the preceding claims, **characterised in that** the contour of the first line of weakness (8) is incomplete so as to retain the part of the film outlined by the first line of weakness attached to the rest of the wrapping after opening.

12. Condom wrapping as in claims 1 to 10, **characterised in that** the contour of the first line of weakness (8) is complete so as to assure the separation of the part of the film outlined by the first line of weakness after opening.
